# EUROPEAN PATENT SPECIFICATION

(11) **EP 3 153 849 B1**
(45) Date of publication and mention of the grant of the patent: **01.12.2021**
(21) Application number: 15188284.2
(22) Date of filing: 05.10.2015
(51) Int. Cl.: G01N 27/12, G01N 33/00

(54) **GAS SENSOR AND METHOD FOR OPERATING SAID GAS SENSOR**
GASSENSOR UND VERFAHREN ZUM BETREIBEN DIESES GASSENSORS
CAPTEUR DE GAZ ET PROCÉDÉ POUR FAIRE FONCTIONNER CE CAPTEUR DE GAZ

(43) Date of publication of application: 12.04.2017
(73) Proprietor: Sensirion AG, 8712 Stäfa (CH)
(72) Inventor: Röck, Frank, 8712 Stäfa (CH); Bartsch, Sebastian, 8712 Stäfa (CH); Höhne, Felix, 8712 Stäfa (CH)

(56) References cited:
- EP-A1- 2 762 868
- EP-A1- 2 778 667
- DE-A1- 4 305 934
- JP-A- S6 031 049
- JP-A- 2013 113 621

## Description

### Field of the Invention

The present invention relates to a method for operating a gas sensor, and to a gas sensor.

### Background of the Invention

Metal-oxide gas sensors are based on the concept that gaseous analytes interact with a metal oxide sensitive layer at elevated temperatures of the sensitive layer in the range of more than 100 °Celsius, and specifically between 250 °C and 450 °C. As a result of a catalytic reaction, a conductivity of the sensitive layer may change which change can be measured. Hence, such gas sensors are also denoted as high temperature chemoresistors for the reason that a chemical property of the analyte is converted into an electrical resistance at high temperatures of the sensitive layer.

The operation of the heater, however, is a main reason for a degradation of the gas sensor in the long-term. Contaminant gases in the atmosphere, such as siloxanes, hydrogen sulfide, hydrocarbons, chlorine, humidity, etc. increasingly act on the sensitive layer the more the sensitive layer is heated. In the light of the above, it is therefore seen as an object of the invention to improve a gas sensor and a method for operating a gas sensor.

JP S6031049 A discloses an atmosphere detecting apparatus with the purpose to enable durable and automatic detection of gas atmosphere, by installing a plurality of atmosphere sensor elements, switching means allowing these elements to act after consecutive switching and judging means for atmosphere responding to the sensor. Semi-fixed resistances VR1-VR4 are connected in series to sensible bodies S1-S4 respectively and the individual resistance, in response to resistance value of the sensible bodies S1-S4 in a concentration at the start of alarm is adjusted in such a way that each output voltage V1-V4 becomes equal. Upon operating interlinked changeover switches SW1, SW2, by heaters H1-H4 at the initial stage of receiving a current, the sensible bodies S1-S4 are heated and an output voltage VS is issued. This VS is delivered to a discriminating circuit for the gas atmosphere. By setting life of the gas detection element in such a way that, the count of generations of output signal VO at its predetermined value causes automatic switching to the following detecting element, the element, upon its reaching the expiration of its life, can automatically cause the succeeding detecting element to operate.

DE 4305934 A1 discloses an arrangement of sensors for measuring air humidity.

EP 2762868 A1 discloses a diffusion based metal oxide gas sensor.

JP 2013113621 A discloses a gas detection apparatus.

EP 2778667 A1 discloses a multi-temperature CMOS gas sensor.

### Summary of the Invention

According to a first aspect of the invention, a method as set out in independent claim 1 is provided for operating a gas sensor. The gas sensor comprises a set of gas sensing cells, each gas sensing cell comprising on or integrated in a substrate: a gas sensitive element and a heater for heating the assigned gas sensitive element. During a first period in the lifetime of the gas sensor, gas measurements are conducted with a first gas sensing cell of the set by activating the corresponding heater to heat the assigned sensitive element, while at the same time the heater of at least one other gas sensing cell of the set remains inactive or is activated solely for conducting reference gas measurements at a rate less than the conducting of the gas measurements by the first gas sensing cell. In response to a trigger, gas measurements are conducted during a second period in the lifetime of the gas sensor following the first period with the other gas sensing cell by activating the corresponding heater to heat the assigned sensitive element, while at the same time the heater of the first gas sensing cell remains inactive or is activated solely for conducting recovery gas measurements at a rate less than the conducting of the gas measurements by the other gas sensing cell.

According to another aspect of the present invention, a gas sensor as set out in independent claim 9 is provided. A control unit of the gas sensor is configured to individually control the heaters of the set of gas sensing cells, and to perform the above method steps.

A gas sensing cell of the gas sensor is understood as unit that comprises an individual sensitive layer and an individual heater assigned for heating the assigned sensitive layer. In addition, it is preferred, that each gas sensing cell comprises its individual set of electrodes for sensing a parameter of the sensitive layer, such as its electrical resistance. The gas sensor preferably is an integrated gas sensor, hence, its elements are integrated on or in a substrate. The substrate preferably is a semiconductor substrate, and preferably is a silicon substrate. Preferably, layers are provided thereon such as insulation and / or metal layers, preferably in form of a CMOS stack of layers. The gas sensitive layer - also referred to as sensitive layer - is arranged on or is integrated in the layers or the substrate. It is noted that the term "arranged on the substrate" shall include both a direct arrangement on a surface of the substrate and an indirect arrangement on the substrate with one or more layers in between. The sensitive layer is sensitive to one or more analytes, also referred to as substances or compounds, for example in a gas to which the gas sensor is exposed to. In a preferred embodiment, the, and preferably each of the sensitive layers comprises a metal-oxide material that gaseous analytes interact with at elevated temperatures of the sensitive layer, e.g. in the range of more than 100 °C, and specifically between 250 °C and 450 °C. As a result of a catalytic reaction, a conductivity of the sensitive layer may change which change is measured. Applications of the gas sensor may include the detection of one or more of toxic gases, ethanol, H2, sulphide compounds or other components in a user's breath, or the detection of other substances.

The heater may be a resistive heater which generates heat upon the application of an electric current, and which may be formed from a metal layer of the stack of layers arranged on the substrate. The electrodes may be formed from a metal layer of the stack of layers, too.

In a preferred embodiment, the gas sensor comprises a membrane spanning a recess in the substrate. Given that the heater and the sensitive element of a gas sensing cell are preferably arranged on or integrated in the membrane, the thermal impact of the heater on the rest of the substrate can be minimized given that the membrane serves for thermally insulating the areas heated by the heater from the rest of the substrate and possibly circuitry that may be integrated in the substrate in a region outside the membrane.

In a preferred embodiment multiple gas sensing cells are implemented on a common substrate. This embodiment is referred to as a single chip gas sensor. Such gas sensor preferably comprises a set comprising at least two gas sensing cells, preferably two, or three, or four gas sensing cells. The individual gas sensing cells may be arranged on a common membrane. While in one embodiment, the membrane may be a continuous membrane without any perforations, in a different embodiment the membrane may have perforations separating the membrane into multiple portions thereof, each portion supporting a dedicated gas sensing cell including the corresponding sensitive element and heater. It is common to both alternatives that the membrane - or the portions thereof - span a common recess in the substrate wherein the continuous membrane may completely cover the recess while the portions each may bridge the recess. The recess may be etched from a back side of the substrate and hence the recess reaches through the substrate with the membrane or the portions thereof being built from at least some layers of the layer stack. In another variant, the substrate is etched from its front side, and is not necessarily etched through, such that the recess may take the form of a cavity once the membrane spans the recess.

In a different embodiment, multiple membranes are provided, e.g. a single one for each gas sensing cell, although implemented on the same chip, i.e. in combination with a common substrate. Here, the membranes are considered to be multiple membranes given that each membrane spans a different recess in the common substrate. In a further embodiment, the various gas sensing cells are not embodied in a common substrate but on multiple individual substrate pieces, for example, each supporting an individual gas sensing cell. Such multiple dies then may preferably be packaged together, including arranging the dies on a common carrier such as a lead-frame, and at partially encapsulating the arrangement e.g. by a mould compound for forming a gas sensor.

The single chip embodiment of the gas sensor may also be packaged, including arranging the chip on a carrier and partially encapsulate this arrangement e.g. with a mould compound.

The gas sensor may be a gas sensor for detecting one or more substances in a gas, and specifically in air surrounding a portable multi-purpose electronic device such as a mobile phone or a tablet computer the gas sensor is arranged in. Hence, in a sample application it may be of interest to identify, if such air may contain analytes the gas sensor is tuned to detect.

According to the invention, each gas sensing cell of the gas sensor comprises the same gas sensitive element, which preferably comprises the same metal-oxide material. All the gas sensing cells of the set - which not necessarily need to be all gas sensing cells of the gas sensor - have the same gas detecting properties such that when one of the gas sensing cells is detected to be degraded or is assumed to be degraded, e.g. owed to the effect of heating, it can be switched from the gas sensing cell presently used first for conducting gas measurements to another gas sensing cell of the set for continuing conducting gas measurements. Hence, out of the set of gas sensing cells, a first gas sensing cell is defined to be the one to conduct gas measurements with, e.g. when starting operating the gas sensor. There may also be multiple first gas sensing cells provided for conducting gas measurements e.g. for different analytes at the same time. After having started operation of the gas sensor, gas measurements are conducted by way of this first gas sensing cell. This means that once a gas measurement is requested, e.g. triggered by a user or by an application, the heater of this first gas sensing cell is activated, and simultaneously or in response to the activation of the heater, a measurement of e.g. a resistance of the heated sensitive layer is taken, which measured value may indicated the presence of an analyte. The gas measurements preferably are performed by the first gas sensing cell throughout a first period in the lifetime of the device, which first period in time may not need to be defined up-front. However, such first period may rather be in the scale of days, or months, than of minutes or seconds.

A trigger - embodiments of which will be described later in more detail - may terminate the first period, and thus may terminate taking gas measurements by this first sensing cell. In response to the trigger, gas measurements will be conducted by another gas sensing cell of the gas sensor. Hence, the conducting of gas measurements is switched from the first gas sensing cell to the other gas sensing cell. The trigger may be generated in response to a criterion being fulfilled, which criterion either assumes or measures at least a beginning degradation of the first gas sensing cell.

Given that the other gas sensing cell is assumed to be "fresh", i.e. not showing degradation, the other gas sensing cell may take over from the first gas sensing cell which presumably is at least near to degradation.

For not straining the other gas sensing cell during the first period, it is preferred to have it retained inactive during this first period. It may be envisaged that the other gas sensing cell is not heated at all during the first period, since the heating is assumed to be responsible for degrading effects. Hence, precise gas measurements can be taken even after the first gas sensing cell suffers from the heat impact given that a new unused gas sensing cell is used thereafter for conducting gas measurements with. The same effect can be achieved, and hence the other gas sensing cell can be considered as still "fresh", if it was only activated occasionally during the first period. Specifically, during the first period, the heater of the other gas sensing cell may solely be used for conducting reference gas measurements at a rate less than the conducting of gas measurements by the first gas sensing cell. Such reference gas measurement may result in a value, such as a resistance value that may be compared to the resistance value measured with the first gas sensing cell for detecting degradation of the first gas sensing cell. For this purpose the other gas sensing cell is desired to remain "fresh" during the entire first period which requires only an occasional use, e.g. for such reference gas measurements. In any case, it is preferred that the reference gas measurements are initiated at a rate less that the rate the actual gas measurements are taken by the first gas sensing cell. Preferably, the rate of the reference gas measurements, and hence the occasions the heater of the other gas sensing cell is activated, is less than a tenth of the rate of the gas measurements conducted with the first gas sensing cell, and preferably less than a hundredth, and preferably less than a thousandth.

The other gas sensing cell - conserved by the measure of not being used at all during the first period or only at a low rate - then is responsible for conducting gas measurements during the second period in the lifetime of the gas sensor, again, which second period is not necessarily defined up-front but may be limited by the generation of another trigger indicating the degradation of this other gas sensing cell. During this second period, the first gas sensing cell preferably is no longer activated, given that gas measurements are now taken by the other gas sensing cell, except that occasional measurements may be taken in order to determine if the first gas sensing cell has already recovered from degradation, which measurements are referred to as recovery gas measurements. For the rate for the recovery gas measurements preferably the same figures apply as for the above introduced reference gas measurements.

As far as the trigger is concerned, it is generated in a first example, which does not form part of the present invention, after a pre-defined time has passed after starting operation of the gas sensor. Hence, the first period indeed is predefined in this example. Assuming an average use of the gas sensor, historic data may indicate at which point in time degradation of the first gas sensing cell may suggest switching gas measurements from the first gas sensing cell to the other gas sensing cell of the set. In a different example, which does not form part of the present invention, heating times of the heater of the first gas sensing cell are accumulated, and if the accumulated heating time exceeds an assigned threshold, the trigger is generated for switching from the first gas sensing cell to the other gas sensing cell for conducting gas measurements. Given that a heating pulse typically is of a defined known period, a counter may be used for counting the number of times a gas measurement is taken by the first gas sensing cell which number may be multiplied by the length of a heating pulse. Here, rather than assuming an average number of gas measurements per time unit, the actual heating time is taken into consideration which is considered to be direct proportional to the degrading of the sensitive material. In another example, which does not form part of the present invention, in addition to the accumulated heating time, the trigger is issued dependent on a temperature or power the heater is heated to or heated with respectively. The higher the temperature the heater is heated to, the more degradation can be observed. For example, in case of applying different heating patterns to the heater a product of heating time by corresponding heating temperature may be calculated for each heating period, and the accumulated products may be compared to a threshold.

In a further class of triggering, not forming part of the present invention, the measurand itself allows for an assessment if the sensitive layer starts degrading or not. The electrical resistance of the sensitive element measured during or in response to heating the sensitive element is used, also e.g. in combination with electrical resistances measured in the past in the first period. For example, the trigger is generated, as soon as an average of values measured so far during the first period falls below or exceeds an assigned threshold; or, alternatively, if a moving average of values measured in the first period falls below or exceeds an assigned threshold. Typically, the value of the measured electrical resistance will increase or decrease over time owed to degradation, which best is detected by averaging the resistance values of the past. The threshold may be set to a value that deviates from a normal average resistance value expected for a non-degraded gas sensing cell.

In a different approach, it is a standard deviation or a moving standard deviation of the electrical resistance values measured during the first period that is to fall below or is to exceed an assigned threshold for causing the generation of the trigger. Again, typically the value of the standard deviation of the measured values will decrease over time owed to degradation, which best is detected by determining the standard deviation from the measured values. The threshold may be set to a value deviating from a normal standard deviation value expected for a non-degraded gas sensing cell.

In a further approach a portion of high-frequency changes in the electrical resistance values measured falls under an assigned threshold. The aforementioned threshold can be understood, e.g. as a cut-off frequency, which decreases with ongoing degradation.

In a different class of triggering, it is not only the measured resistance values preferably including those of the past that are used for determining the trigger event. It is also, according to the present invention, a change in the measured electrical resistance that is monitored in response to activating the heater, preferably by applying a current in a step function of defined amplitude. If such change exceeds an assigned threshold, the trigger is generated. In addition, the trigger is generated if a period in time for the measured electrical resistance value to reach a target value in response to activating the heater exceeds an assigned threshold.

In another class of triggering, as set out in the dependent claims, the measured values are compared with reference values measured with another, non-degraded gas sensing cell, which in one embodiment may be a gas sensing cell of the set used throughout the lifetime of the gas sensor as reference gas sensing cell only. In this embodiment, the set contains at least three gas sensor cells, wherein during the lifetime of the gas sensor the heater of the third, reference gas sensing cell of the set remains inactive except for conducting reference gas measurements to be compared to results of gas measurements conducted with any of the first gas sensing cell or the one or more other gas sensing cells. Again, the rate of the reference gas measurements is far less than the rate of operative gas measurements, such as at rates defined above. The at least one third gas sensing cell is used only for reference gas measurements which may be conducted at intervals such as per days that cumulatively do not impact the quality of the third gas sensing cell throughout the lifetime of the gas sensor.

In a different embodiment, the reference gas sensing cell is identical to the other gas sensing cell. Hence, occasionally reference gas measurements are performed by the other gas sensing cell, and once a degradation is detected for the first gas sensing cell dependent on the result of the reference gas measurement, the gas measurements may be conducted by the other sensor cell that during the first period also acted as reference gas sensing cell, but during the second period becomes the operative gas sensing cell.

In those embodiments, the trigger may be issued dependent on a value measured during a gas measurement and dependent on a reference value measured during a reference gas measurement, wherein specifically the trigger is issued when the measured value deviates from the measured reference value by more than an assigned margin.

In a different embodiment including a reference gas sensing cell, a change in the electrical resistance in response to activating the heater of the first gas sensing cell is compared to a change in the electrical resistance measured in response to activating the heater of the reference gas sensing cell, and the trigger is issued when those change values deviate from each other by more than an assigned margin. In a different embodiment, a period in time for the measured value to reach a target value in response to activating the heater of the first gas sensing cell deviates from a period in time for the measured reference value to reach the target value in response to activating the heater of the reference gas sensing cell by more than an assigned margin.

In a further embodiment, an additional sensor is provided, and preferably integrated into the same substrate as the gas sensor, or packaged together with the gas sensor. This additional sensor preferably measures a different measurand than the gas sensor. In one embodiment the additional sensor is a humidity sensor, while the gas sensor is not designed to explicitly measure humidity. The trigger may now depend on both the result of the gas measurement taken by the currently used gas sensing cell, and by a measurement result provided by the additional sensor. Typically, the measurement results, which in both cases may be electrical resistances, are correlated. In case the correlation exceeds an assigned threshold, the trigger may be generated.

Generally, it is preferred that more than one other/redundant gas sensing cell is provided, and preferably an array of two, three or more other gas sensing cells. In the event of at least two other gas sensing cells, the same method applied for switching from the first gas sensing cell to the other gas sensing cell may be applied to further switch from the other gas sensing cell, now referred to as first other gas sensing cell, to a second other gas sensing cell. In response to another trigger indicating the beginning or an existing degradation of the first other gas sensing cell, the conducting of gas measurements is switched from the first other gas sensing cell to the second other gas sensing cell. The other trigger may be of the same nature as the first trigger, however, now with respect to the first other gas sensing cell. In the following, gas measurements are conducted only with the second other gas sensing cell for a third period in the lifetime of the gas sensor which follows the second period. During this third period, the heaters of the previously used gas sensing cells are preferably not activated. There may be even more other gas sensing cells provided in the set. The control unit then may switch from one other degraded gas sensing cell to the next one in response to an appropriate trigger.

In a different variant, in response to a further trigger, gas measurements may be switched from a degraded other gas sensing cell to one of the gas sensing cells previously used, e.g. in particular to the first gas sensing cell. This first gas sensing cell may regenerate while not being used for gas measurements and get back its quality measurement results, which process is referred to as recovery.

The control unit for controlling the activation / deactivation of individual gas sensing cells may be integrated in the common substrate in case of the gas sensing cells being integrated in such common substrate. Hence, such single chip gas sensor may additionally include the control unit and appropriate circuitry for switching between gas sensing cells for conducting gas measurements. In addition, means may be provided, e.g. in one or more of hardware, firmware, software, for executing a gas measurement routine. The gas measurement routine preferably is defined by a heating process over time and by one or more measurement points in time. In case of a multiple die or chip embodiment, each chip may contain local means for executing its own gas measurement routine, while another die or chip may be provided for implementing the control unit for executing the switching routine. Hence, the control unit triggers gas measurements routines at the various dies incorporating gas sensing cells as desired.

It is noted that embodiments described in relation to one category of claims shall be considered as disclosed in connection with the one or more other category, too.

Other advantageous embodiments are listed in the dependent claims as well as in the description below.

### Brief Description of the Figures

The embodiments defined above and further aspects, features and advantages of the present invention can also be derived from the examples of embodiments to be described hereinafter and are explained with reference to the drawings. In the drawings the figures illustrate in
FIG. 1 a gas sensing cell as used in a gas sensor according an embodiment of the invention, in a perspective view the gas sensing cell being cut open in diagram 1A; and a side cut of a portion of the membrane of the gas sensing cell of diagram 1A, in diagram 1B;
FIG. 2 a gas sensor according to an embodiment of the present invention, in a top view in diagram 2A, and in a cut along A-A' in diagram 2B;
FIG. 3 a gas sensor according to another embodiment of the present invention, in a top view in diagram 3A, and in a cut along B-B' in diagram 3B;
FIG. 4 a schematic diagram of a gas sensor according to an embodiment of the present invention at the same time illustrating a method for controlling the gas sensor according an embodiment of the present invention;
FIG. 5 a schematic diagram of a gas sensor according to another embodiment of the present invention at the same time illustrating a method for controlling the gas sensor according another embodiment of the present invention;
FIG. 6 a schematic diagram of a gas sensor according to a further embodiment of the present invention at the same time illustrating a method for controlling the gas sensor according a further embodiment of the present invention;
FIG. 7 a flow chart of a method for operating a gas sensor according to an embodiment of the present invention.

### Detailed Description of the Figures

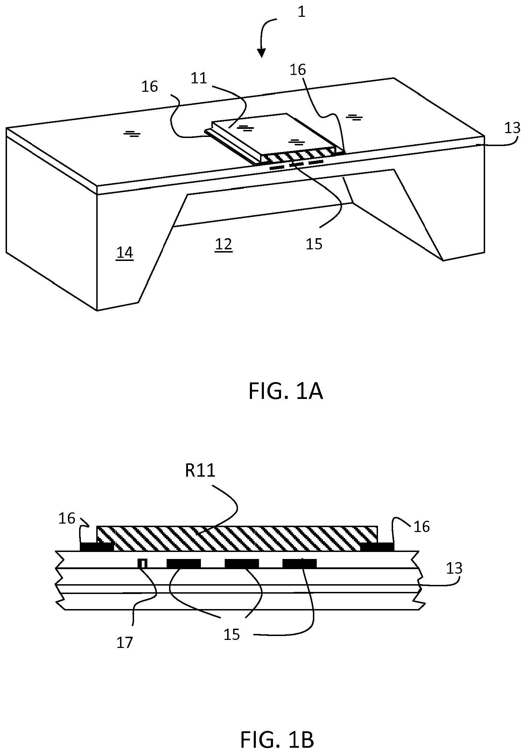

Figure 1 illustrates a gas sensing cell of a gas sensor according to an embodiment of the present invention, in a perspective view in diagram 1A, and in a cut through of a portion of the membrane in diagram 1B. The gas sensing cell is an integrated gas sensing cell 1 comprising a substrate 14 such as a silicon substrate. Layers, e.g. CMOS layers, are arranged on the substrate 14. A portion of the substrate 14, and possibly a portion of the layers are etched away to form a cavity 12. The remaining layers, and possibly a portion of the substrate 14 contribute to a membrane 13 spanning the recess 12. A gas sensitive layer 11 is arranged at a location of the membrane 13 spanning the recess 13. The gas sensitive layer 11 preferably comprises metal oxide material and is sensitive to one or more gas compounds. The membrane 13 therefore supports the sensitive layer 11. Embedded within the membrane 13 are conducting elements forming a heater 15 to provide a local source of heat to heat the metal oxide sensitive layer 11 during operation of the gas sensing cell 1. In response to heating the heater 15, the membrane 13 acts as a hot plate and temperature can rise rapidly around the metal oxide sensitive layer 11, while the remaining substrate 14 reacts due to its thermal inertia with a slower rise of temperature. By controlling the heater 15 accordingly, a chemical reaction in the vicinity of the hotplate can be activated, which can be detected by the metal oxide sensitive layer 11.

The metal oxide sensitive layer 11 is contacted by two conductive electrodes 16 and hence acts as a resistor R11. In the presence of an analyte this resistance R11 changes thereby providing a measure of the concentration of the analyte in the immediate vicinity of the metal oxide sensitive layer 11.

In one embodiment, an additional temperature sensor 17 may be integrated on or into the membrane for measuring a temperature thereof. A polycrystalline silicon (poly-Si) or a metal resistor can be used as temperature sensor, for example.

The gas sensing cell 1 may be integrated with a CMOS circuitry (not shown) which CMOS circuitry may encompass at least a memory, a system controller, an engine for performing a measurement routine stored in the memory, and other circuitry.

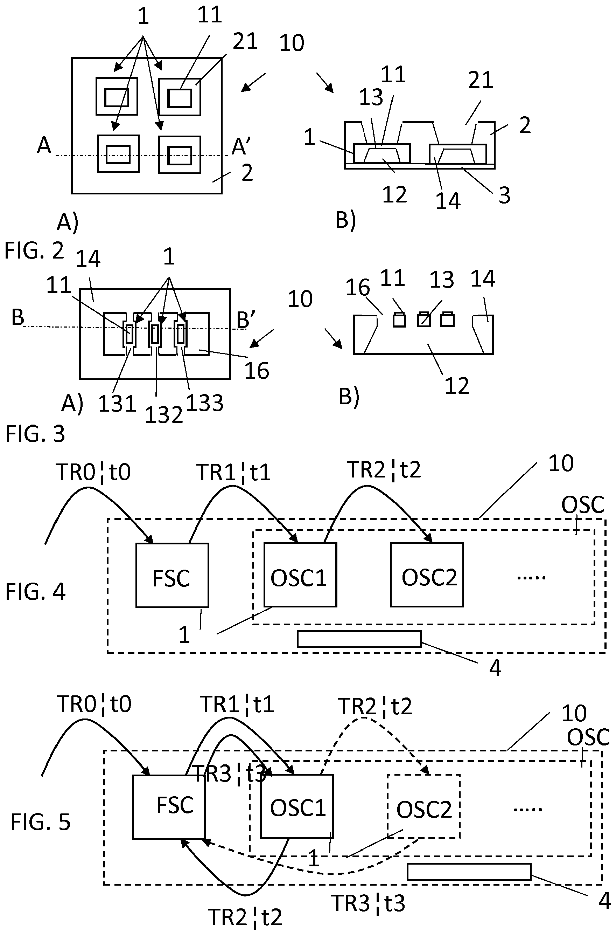

Figure 2 illustrates a gas sensor 10 according to an embodiment of the present invention, in a top view in diagram 2A, and in a cut along A-A' in diagram 2B. The gas sensor 10 of the present example is a gas sensor package containing multiple individual gas sensing cells 1, e.g. according to Figure 1, packaged together. Presently, there are four gas sensing cells 1 packaged into a single gas sensor 10, however, the number as well as the arrangement in the package may vary. Each gas sensing cell 1 shows, similar to the gas sensing cell of Figure 1, a substrate 14 containing a recess 12, a membrane 13 spanning the recess 12, and a sensitive element 11 arranged on or integrated into the membrane 13. As can be derived from diagram 2B, the gas sensing cells 1 are arranged on a common carrier 3, such as a lead-frame. The gas sensing cells 1 are partly encapsulated by e.g. a mould compound 2 having openings 21 for granting access to the sensitive elements 11 for the medium to sense. It is preferred, that the gas sensing cells 1 of the present gas sensor 10 are identical with respect to the sensing capabilities, i.e. all may have the same sensitive element 11, including the same material of the sensitive element 11, all may have the same filter, if any, for filtering the medium to be sensed prior to reaching the sensitive elements 11, etc. Hence, it can be assumed that each of the gas sensing cells 1 would provide the same signal in response to a gas compound applied, provided there is no degradation.

In the embodiment of Figure 2, each gas sensing cell 1 may comprise an individual circuitry integrated, which circuitry may include means for controlling the heater, e.g. for selectively applying a heating current to the heater for a defined period in time. The circuitries of each gas sensing cell may in turn be controlled by a control unit that may be embodied as an individual integrated circuit, such as an ASIC, which may be arranged on the same carrier 3 and be packaged with the same mould compound 3.

Figure 3 illustrates a gas sensor 10 according to another embodiment of the present invention, in a top view in diagram 3A, and in a cut along B-B' in diagram 3B. The gas sensor 10 of the present example is a gas sensor containing multiple individual gas sensor cells 1 in a common substrate 14. Presently, there are three gas sensing cells 1 provided in the common substrate 14, however, the number and the design may vary. Presently, the gas sensor 10 is not packaged. However, in a different embodiment, the gas sensor of Figure 3 may also be packaged, and e.g. be arranged on a carrier such as a lead-frame, and be partly encapsulated except for an access opening to the sensitive elements 11. A membrane 13 spanning a common recess 12 in the substrate 14 comprises three portions 131, 132, 133. Each of the sensitive elements 11 is arranged on an assigned portion of the membrane 13 spanning the common recess 12 in the substrate 14. The membrane 13 may be formed from a stack of layers, such as CMOS layers arranged on the substrate 14 prior to etching the recess 12. While in a different embodiment, a continuous membrane may span the entire recess 12, in the present embodiment, the membrane is provided with perforations 16, e.g. manufactured by etching from the continuous membrane, such that in the present example, bridge like structures act as portions 131, 132, 133 of the membrane. Each portion 131, 132, 133 of the membrane comprises an assigned sensitive layer 11 arranged thereon or integrated therein, and a dedicated heater (not shown), e.g. arranged from metal layers in the membrane portion for heating the assigned sensitive element 11. In addition, each membrane portion 131, 132, 133 preferably comprises electrodes for contacting the sensitive layer 11 (not shown).

The gas sensor 10 of Figure 3 may comprise circuitry integrated into the common substrate 14. This integrated circuitry preferably controls the heaters of the various gas sensing cells 1 individually - i.e. independent from each other, e.g. by applying heating currents at defined periods in time, and controls the switching between the gas sensing cells.

Figure 4 illustrates a schematic diagram of a gas sensor according to an embodiment of the present invention at the same time illustrating a method for controlling the gas sensor according an embodiment of the present invention. The gas sensor 10 comprises a set of gas sensing cells 1, each schematically illustrated by a rectangle. The set comprises a first gas sensing cell FSC, and a subset of N other redundant gas sensing cells OSC, including a first other gas sensing cell OSC1, a second other gas sensing cell OSC2, and possibly further other gas sensing cells indicated by dots. In the present embodiment, in response to a trigger TR0 at time t0, the first gas sensing cell FSC is assigned for conducting gas measurements. The trigger TR0 in this event may, for example, be the starting of operation of the gas sensor. At some point in time t1, it may be found that the first gas sensing cell FSC no longer provides measurements of the desired quality. A trigger TR1 issued at such time t1 indicates this scenario and causes switching the conducting of gas measurements from the first gas sensing cell FSC to the first one of the other gas sensing cells OSC1, i.e. the first redundant gas sensing cells OSC1 out of the set of other gas sensing cells OSC, also referred to as backup gas sensing cells. The time t1 - t0 = T1 represents the first period T1 in the lifetime of the gas sensor 10 the first gas sensing cell FSC was active for taking gas measurements. In the following, i.e. during a second period T2 in the lifetime of the gas sensor 10, gas measurements are taken by the first other gas sensing cell OSC1.

A trigger TR2 issued at time t2 indicates that at this point in time the first other gas sensing cell OSC1 no longer provides measurement results of the desired quality which trigger TR2 causes switching the conducting of gas measurements from the first other gas sensing cell OSC1 to the second other gas sensing cell OSC2. The time t2 - t1 = T2 represents the second period T2 in the lifetime the first other gas sensing cell OSC1 was active for taking gas measurements. In the following, gas measurements are taken by the second other gas sensing cell OSC2. This concept can be continued subject to the number N of other gas sensing cells OSC being available in the gas sensor 10.

Figure 4, as well as Figures 5 and 6 illustrate a control unit 4, preferably integrated into the gas sensor 10, which control unit 4 automatically executes the processes as described.

Figure 5 illustrates a schematic diagram of a gas sensor according to another embodiment of the present invention at the same time illustrating a method for controlling the gas sensor according anther embodiment of the present invention. The gas sensor 10 comprises the same set of gas sensing cells 1 as in Figure 4. The method applied is identical to the method explained in connection with Figure 4 until time t2. At time t2, it is again detected that the first other gas sensing cell OSC1 becomes degraded, such that switching to another gas sensing cell may be desired for the further conducting of gas measurements.

However, now it is not switched to the second other gas sensing cell OSC2 but back to the first gas sensing cell FSC which may have recovered from degradation in the meantime. Such recovery may be defined by a sufficient time having passed since the deactivation of the first gas sensing cell at time t1. Hence, in case the second period T2 is considered as sufficient long for allowing a recovery of the first gas sensing cell FSC, a bare timer may cause switching back to the first gas sensing cell FSC. In a different embodiment, the subject first gas sensing cell FSC may be investigated if it already has recovered at time t2. For example, measurements are taken at that point in time t2 with the first gas sensing cell FSC, e.g. by one of the methods illustrated above for detecting a degradation of a gas sensing cell.

Accordingly, at time t2, the first other gas sensing cell OSC1 is detected to start degrading, and the first gas sensing cell FSC is detected to have recovered from degradation, such that a fulfilling of these two requirements triggers a switching of taking gas measurements from the first other gas sensing cell OSC1 back to the first gas sensing cell FSC. After a third period in time t3 - t2 = T3, i.e. at time t3, the first gas sensing cell FSC may be detected as degraded again, such that this trigger TR3 switches the conducting of gas measurements from the first gas sensing cell FSC to the first other gas sensing cell OSC1 again, provided the first other gas sensing cell OSC1 is detected to have recovered at this point in time t3.

In a different variant indicated by the dashed lines, at time t2 the first gas sensing cell FSC is not detected to have recovered yet. In this scenario, the trigger TR2 causes switching the taking of gas measurements from the first other gas sensing cell OSC1 to the second other gas sensing cell OSC2. For example, only at time t3, it may be detected that the first gas sensing cell FSC has recovered, such that in response to detecting a degradation in the second other gas sensing cell OSC2, it is switched back to the first gas sensing cell FSC.

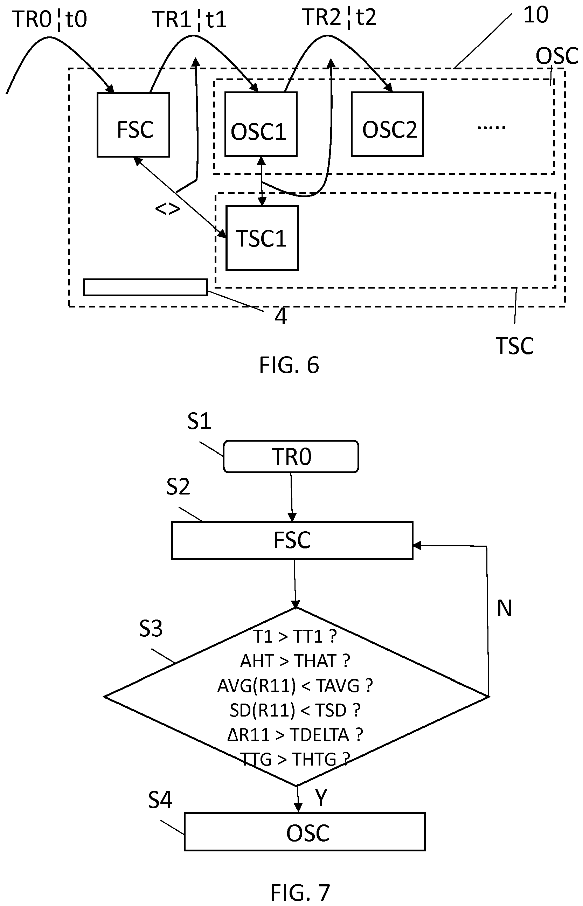

Figure 6 illustrates a schematic diagram of a gas sensor according to a further embodiment of the present invention at the same time illustrating a method for controlling the gas sensor according a further embodiment of the present invention. The gas sensor 10 again comprises a set of gas sensing cells 1, each schematically illustrated by a rectangle. The set comprises a first gas sensing cell FSC, and a subset of N other redundant gas sensing cells OSC, including a first other gas sensing cell OSC1, a second other gas sensing cell OSC2 ... In addition a subset of third gas sensing cells TSC is provided in the gas sensor, also referred to as reference gas sensing cells, containing in the present example only one third gas sensing cell TSC1. The third gas sensing cell TSC is identical to the first and the other gas sensing cells FSC, OSC. The method applied is identical to the method explained in connection with Figure 4. In response to a trigger TRx, gas measurements are no longer conducted with the present gas sensing cell but with one of the other gas sensing cells OSC.

In the present example, which does not form part of the present invention, the trigger event is now more elaborated. For generating a trigger TRx, measurement results of the present gas sensing cells, e.g. the first gas sensing cell FSC and the third gas sensing cell TSC are compared. It is assumed that the third gas sensing cell TSC is only used for occasional reference gas measurements to detect a possible degradation of the gas sensing cell presently used for regular gas measurements. Hence, the third gas sensing cell TSC does not suffer from degradation throughout the lifetime of the gas sensor owed to the little heating time accumulated for the reference gas measurements. In the present example, it is always the reference gas sensing cell TSC being used for determining the degradation of the presently used gas sensing cell. Hence, the third gas sensing cell TSC presently contributes to the decision if a trigger is generated or not.

Note, that in a different example, which does not form part of the present invention, one of the other gas sensing cells OSC may take over the function of the third gas sensing cell TSC of Figure 6: One of the other gas sensing cells OSC not being used for conducting gas measurements so far could be used as reference gas sensing cell. The difference compared to the arrangement of Figure 6 is, that this other gas sensing cell may finally also be used for conducting gas measurements, while in the arrangement of Figure 6, the one or more reference gas sensing cells TSC are never used except for conducting the reference gas measurements.

Figure 7 illustrates a flow chart of a method for operating a gas sensor according to an embodiment of the present invention. In step S1, trigger T0 is generated, which e.g. is the first switching on of the gas sensor, which triggers the conducting of gas measurements with the first gas sensing cell FSC for the first period in the lifetime of the gas sensor. In step S3, it is monitored, e.g. at regular intervals, if the first gas sensing cell FSC is subject to degradation, and in particular is already degraded such that gas measurements are better conducted with a replacement gas sensing cell, i.e. another gas sensing cell OSC, as indicated in step S4 for the scenario when such degradation is detected in step S3 (Y). If no such degradation is detected in step S3 (N), gas measurements are continued to be conducted by the first gas sensing cell FSC. In step S3, different sorts of trigger conditions are illustrated: One of these trigger conditions preferably is applied throughout the lifetime of the gas sensor.

In a first variant, which does not form part of the present invention, the trigger is generated if the condition is fulfilled (Y), that the first period T1, i.e. the period gas measurements are taken by the first gas sensing cell FSC, exceeds an assigned threshold TT1.

In a second variant, which does not form part of the present invention, the trigger is generated (Y) as soon as an accumulated heating time AHT exceeds an assigned threshold THAT, which accumulated heating time AHT is represented by the time the heater of the first gas sensing cell FSC is activated so far during the first period T1.

In a third variant, the trigger is generated (Y) as soon as an average, e.g. a moving average AVG of the electrical resistances R11 of the sensitive element of the first gas sensing cell FSC measured falls below an assigned threshold TAVG.

In a fourth variant, the trigger is generated (Y) as soon as a standard deviation, e.g. a moving standard deviation SD of the electrical resistances R11 of the sensitive element of the first gas sensing cell FSC measured falls below an assigned threshold TSD.

In a fifth variant, the trigger is generated (Y) as soon as change in the measured electrical resistance value ΔR11 in response to activating the heater of the first gas sensing cell FSC exceeds an assigned threshold TDELTA. According to the present invention, the fifth variant is combined with the third or fourth variant.

In addition, the trigger is generated (Y) as soon as a period in time TTG for the measured electrical resistance value to reach a target value in response to activating the heater of the first gas sensing cell FSC exceeds an assigned threshold THTG.

## Claims

1. Method for operating a gas sensor, the gas sensor (10) comprising a set of gas sensing cells (1) having the same gas detecting properties, each gas sensing cell (1) comprising on or integrated in a substrate (14) a gas sensitive element (11), a set of electrodes (16), a heater (15) for heating the assigned gas sensitive element, and a control unit (4) for controlling the heaters of the individual gas sensing cells and performing the method steps below, the method comprising
- during a first period (T1) in the lifetime of the gas sensor (10) conducting gas measurements with a first gas sensing cell (FSC) of the set by activating the corresponding heater (15) to heat the assigned sensitive element (11), while at the same time leaving the heater (15) of another gas sensing cell (OSC) of the set inactive or activating this heater solely for conducting a reference gas measurement at a rate less than the conducting of gas measurements by the first gas sensing cell,
- in response to a trigger (TR1), conducting gas measurements during a second period (T2) in the lifetime of the gas sensor (10) following the first period (T1) with the other gas sensing cell (OSC) by activating the corresponding heater (15) to heat the assigned sensitive element (11), while at the same time leaving the heater (15) of the first gas sensing cell (FSC) inactive or activating this heater solely for conducting a recovery gas measurement at a rate less than the conducting gas measurements by the other gas sensing cell,
wherein the conducting of a gas measurement includes measuring an electrical resistance (R11) of the subject gas sensing cell (1) during activating or after having activated the corresponding heater (15), and
wherein the trigger (TR1) is generated by the control unit (4) dependent on one or more of a value of the electrical resistance (R1) measured and a change in the measured electrical resistance value (ΔR1) exceeding an assigned threshold in response to activating the heater (15).

2. Method according to claim 1, comprising
generating the trigger (TR1) dependent on one or more electrical resistance values (R11) measured in the past of the first period (T1), and
in particular generating the trigger (TR1) dependent on one or more:
- an average of electrical resistance values (AVG(R11)) measured so far in the first period (T1) falls below or exceeds an assigned threshold (TAVG);
- a moving average of electrical resistance values measured in the first period (T1) falls below or exceeds an assigned threshold;
- a standard deviation of electrical resistance values (SD(R11)) measured so far in the first period (T1) falls below or exceeds an assigned threshold (TSD);
- a moving standard deviation of electrical resistance values measured in the first period (T1) falls below or exceeds an assigned threshold;
- a portion of high-frequency changes in the electrical resistance values measured.

3. Method according to claim 1, comprising
generating the trigger (TR1) depending on one or more of:
- the change in the measured electrical resistance value (ΔR11) in response to activating the heater (15) exceeds an assigned threshold (TDELTA);
- a period in time (TTD) for the measured electrical resistance value (R11) to reach a target value in response to activating the heater (15) exceeds an assigned threshold (THTG).

4. Method according to any of the preceding claims, comprising
during the first period (T1), leaving the heaters (15) of at least two other gas sensing cells (OSC) of the set inactive,
during the second period (T2) conducting gas measurements with a first (OSC1) of the at least two other gas sensing cells (OSC), while at the same time leaving the heater (15) of a second (OSC2) of the at least two other gas sensing cells (OSC) inactive,
in response to another trigger (TR2) conducting gas measurements during a third period (T3) in the lifetime of the gas sensor (10) following the second period (T2) with the second (OSC2) of the at least two other gas sensing cells (OSC), while at the same time leaving the heater (15) of the first gas sensing cell (FSC) and the first (OSC1) of the at least two other gas sensing cells (OSC) inactive.

5. Method according to any of the preceding claims, comprising
in response to a further trigger (TR3) conducting gas measurements during a later period in the lifetime of the gas sensor (10) at least after the second period (T2) with the first gas sensor cell (FSC) again, while at the same time leaving the heater (15) of the at least one other gas sensing cell (OSC) inactive.

6. Method according to any of the preceding claims, comprising
during the lifetime of the gas sensor (10) leaving the heater (15) of a third gas sensing cell (TSC) of the set inactive except for conducting reference gas measurements, and
comparing a result of the gas measurement conducted with any of the first gas sensing cell (FSC) or the at least one other gas sensing cells (OSC) with a result of the reference gas measurement conducted with the third gas sensing cell (TSC).

7. Method according to claim 6, comprising
the conducting of a gas measurement includes measuring an electrical resistance (R11) of the subject gas sensing cell (FSC, OSC) during activating or after having activated the corresponding heater (15), and
generating the trigger (TR1) dependent on an electrical resistance value measured during the gas measurement and dependent on a reference electrical resistance value measured during the reference gas measurement, and
in particular generating the trigger (TR1) depending on one or more of:
- the measured value deviates from the measured reference value by more than an assigned margin;
- a change in the measured value in response to activating the assigned heater deviates from a change in the measured reference value in response to activating the assigned heater by more than an assigned margin;
- a period in time for the measured electrical resistance value to reach a target value in response to activating the assigned heater deviates from a period in time for the measured reference value to reach the target value in response to activating the assigned heater by more than an assigned margin.

8. Computer readable element comprising computer readable code means for performing the steps of a method according to any one of the preceding claims when executed on a control unit of a gas sensor according to claims 9-11.

9. A gas sensor (10), comprising:
a set of gas sensing cells (1) having the same gas detecting properties, each gas sensing cell (1) comprising on or integrated in a substrate (14):
a gas sensitive element (11),
a set of electrodes (16),
a heater (15) for heating the assigned gas sensitive element (11),
a control unit configured to individually control the heaters (15) of the set of gas sensing cells (1),
wherein the control unit is configured to, during a first period (T1) in the lifetime of the gas sensor (10), conduct gas measurements with a first gas sensing cell (FSC) of the set by activating the corresponding heater (15) to heat the assigned sensitive element (11) while at the same time leaving the heater (15) of at least one other gas sensing cell (OSC) of the set inactive or activating this heater solely for conducting a reference gas measurement at a rate less than the conducting of gas measurements by the first gas sensing cell,
wherein, in response to a trigger event (TR1), the control unit is configured to, during a second period (T2) in the lifetime of the gas sensor (10) following the first period (T1), conduct gas measurements with the other gas sensing cell (OSC) by activating the corresponding heater (15) to heat the assigned sensitive element (11), while at the same time leaving the heater (15) of the first gas sensing cell (FSC) inactive or activating this heater solely for conducting a recovery gas measurement at a rate less than the conducting of gas measurements by the other gas sensing cell,
wherein the control unit is configured to generate the trigger (TR1) dependent on one or more of a value of the electrical resistance (R1) measured and a change in the measured electrical resistance value (ΔR1) exceeding an as-signed threshold in response to activating the heater (15).

10. Gas sensor (10) according to claim 9,
wherein each gas sensing cell (1) of the set comprises the same gas sensitive element (11), and
wherein each gas sensing cell (1) of the set comprises a metal-oxide material.

11. Gas sensor (10) according to claim 9 or claim 10,
wherein the substrate (14) is a common substrate for all gas sensing cells (1) of the set,
comprising a membrane (13), the membrane (13) comprising separate sub-portions (131, 132, 133) each spanning a common recess (12) in the common substrate ,
wherein each sub-portion (131, 132, 133) supports one of the heaters (15) and the assigned sensitive element (11).

## Patentansprüche

1. Verfahren zum Betrieb eines Gassensors (10), der eine Menge von Gassensorzellen (1) umfasst, die die gleichen Gaserkennungseigenschaften haben, wobei jede Gassensorzelle (1) auf oder integriert in einem Substrat ein gassensitives Element (11), eine Menge von Elektroden (16), einen Heizer zum Heizen des zugeordneten gassensitiven Elements, und eine Steuerungseinheit (4) zur Steuerung der Heizer der individuellen Gassensorzellen und zum Ausführen der folgenden Verfahrensschritte umfasst, wobei das Verfahren umfasst:
- während eines ersten Zeitabschnitts (T1) im Leben des Gassensors (10) mit einer ersten Gassensorzelle (FSC) der Menge Gasmessungen auszuführen durch Einschalten des entsprechenden Heizers (15), um das zugeordnete gassensitive Element (11) zu heizen, und gleichzeitig den Heizer (15) einer anderen Gassensorzelle (OSC) der Menge ausgeschaltet zu lassen oder diesen Heizer nur für das Durchführen einer Referenz-Gasmessung mit einer niedrigeren Rate als das Durchführen der Gasmessung durch die erste Gassensorzelle einzuschalten,
- auf einen Auslöser (TR1) hin Gasmessungen während eines zweiten Zeitabschnitts (T2) im Leben des Gassensors (10) nach dem ersten Zeitabschnitt (T1) mit der anderen Gassensorzelle (OSC) durch Einschalten des entsprechenden Heizers (15) auszuführen, um das zugeordnete gassensitive Element (11) zu heizen, und gleichzeitig den Heizer (15) der ersten Gassensorzelle (FSC) ausgeschaltet zu lassen oder diesen Heizer nur für das Durchführen von einer Wiederherstellungs-Gasmessung mit einer niedrigeren Rate als das durchführen der Gasmessung durch die andere Gassensorzelle einzuschalten,
wobei das Durchführen einer Gasmessung das Messen eines elektrischen Widerstands (R11) der jeweiligen Gassensorzelle (1) während des Einschaltens oder nach dem Einschalten des entsprechenden Heizers (15) beinhaltet, und
wobei der Auslöser (TR1) durch die Steuerungseinheit (4) in Abhängigkeit von mindestens einem von dem Wert des gemessenen elektrischen Widerstands (R1) und der Änderung des gemessenen elektrischen Widerstandswerts (ΔR1) beim Einschalten des Heizers (15), der über eine zugeordnete Schwelle hinausgeht, generiert wird.

2. Verfahren nach Anspruch 1, umfassend das
Generieren des Auslösers (TR1) in Abhängigkeit von mindestens einem gemessenen elektrischen Widerstandswert (R11) im vergangenen Teil des ersten Zeitabschnitts (T1),
und insbesondere das Generieren des Auslösers (TR1) in Abhängigkeit von einem oder mehreren von:
- ob ein Mittelwert von bislang im Zeitabschnitt (T1) gemessenen elektrischen Widerstandswerten (AVG(R11) (T1) unter oder über eine zugeordnete Schwelle (TAVG) fällt;
- ob ein gleitender Mittelwert von im ersten Zeitabschnitt (T1) gemessenen elektrischen Widerstandswerten unter oder über eine zugeordnete Schwelle fällt;
- ob eine Standardabweichung von bislang im ersten Zeitabschnitt (T1) gemessenen elektrischen Widerstandswerten (SD(R11)) unter oder über eine zugeordnete Schwelle (TSD) fällt;
- ob eine gleitende Standardabweichung von im ersten Zeitabschnitt (T1) gemessenen elektrischen Widerstandswerten über oder unter eine zugeordnete Schwelle fällt;
- einem Teil von Hochfrequenzänderungen im gemessenen elektrischen Widerstand,
geschieht.

3. Verfahren nach Anspruch 1, umfassend das Generieren des Auslösers (TR1) in Abhängigkeit von einem oder mehreren von:
- ob die Änderung im gemessenen elektrischen Widerstandswert (ΔR11) beim Einschalten des Heizers (15) eine zugeordnete Schwelle (TDELTA) übersteigt;
- ob ein Zeitabschnitt (TTD), den der gemessene elektrische Widerstandswert (R11) beim Einschalten des Heizers (15) braucht, um einen Zielwert zu erreichen, eine zugeordnete Schwelle (THTG) übersteigt.

4. Verfahren nach einem der vorangehenden Ansprüche, umfassend
während des ersten Zeitabschnitts (T1) den Heizer (15) von mindestens zwei anderen Gassensorzellen (OSC) der Menge ausgeschaltet zu lassen,
während des zweiten Zeitabschnitts (T2) Gasmessungen mit einer ersten Gassensorzelle (OSC1) von den mindestens zwei anderen Gassensorzellen (OSC) auszuführen, und gleichzeitig den Heizer (15) einer zweiten Gassensorzelle (OSC2) von den mindestens zwei anderen Gassensorzellen (OSC) ausgeschaltet zu lassen,
als Reaktion auf einen anderen Auslöser (TR2) Gasmessungen während eines dritten Zeitabschnitts (T3) im Leben des Gassensors (10) nach dem zweiten Zeitabschnitt (T2) mit der zweiten (OSC2) der mindestens zwei anderen Gassensorzellen (OSC) auszuführen, und gleichzeitig den Heizer (15) der ersten Gassensorzelle (FSC) und der ersten anderen (OSC1) der mindestens zwei anderen Gassensorzellen (OSC) ausgeschaltet zu lassen.

5. Verfahren nach einem der vorangehenden Ansprüche, umfassend
als Reaktion auf einen weiteren Auslöser (TR3) Gasmessungen während eines späteren Zeitabschnitts im Leben des Gassensors (10) mindestens nach dem zweiten Zeitabschnitt (T2) wieder mit der ersten Gassensorzelle (FSC) auszuführen, und gleichzeitig den Heizer (15) von der mindestens einen anderen Gassensorzelle (OSC) ausgeschaltet zu lassen.

6. Verfahren nach einem der vorangehenden Ansprüche, umfassend
während des Lebens des Gassensors (10) den Heizer (15) einer dritten Gassensorzelle (TSC) der Menge ausser für Referenz-Gasmessungen ausgeschaltet zu lassen, und
ein Ergebnis der Gasmessung, die mit einer der ersten Gassensorzellen (FSC) oder der mindestens einen anderen Gassensorzelle (OSC) durchgeführt wurde, mit dem Ergebnis einer Referenz-Gasmessung, die mit der dritten Gassensorzelle (TSC) durchgeführt wurde, zu vergleichen.

7. Verfahren nach Anspruch (6), umfassend
das Durchführen einer Gasmessung beinhaltet das Messen eines elektrischen Widerstands (R11) der jeweiligen Gassensorzelle (FSC, OSC) beim oder nach dem Einschalten des entsprechenden Heizers (15), und
das Generieren des Auslösers (TR1) in Abhängigkeit von einem gemessenen elektrischen Widerstandswert während der Gasmessung und in Abhängigkeit von einem gemessenen elektrischen Referenz-Widerstandswert während der Referenz-Gasmessung, und
insbesondere das Generieren des Auslösers (TR1) in Abhängigkeit von einem oder mehreren von:
- der gemessene Wert weicht vom gemessenen Referenzwert um mehr ab als eine zugeordnete Spanne;
- eine Änderung im gemessenen Wert als Reaktion auf das Einschalten des zugeordneten Heizers weicht von der Änderung im gemessenen Referenzwert als Reaktion auf das Einschalten des zugeordneten Heizers um mehr ab als eine zugeordnete Spanne;
- ein Zeitabschnitt, den der elektrische Widerstandswert braucht, um einen Zielwert als Reaktion auf das Einschalten des zugehörigen Heizers zu erreichen, weicht vom Zeitabschnitt, den der elektrische Referenz-Widerstand braucht, um den Zielwert zu erreichen als Reaktion auf das Einschalten des zugeordneten Heizers um mehr als eine zugeordnete Spanne ab.

8. Computer-lesbares Element umfassend computer-lesbare Code-Mittel für das Durchführen der Schritte eines Verfahrens nach einem der vorangehenden Ansprüche, wenn das Verfahren auf einer Steuerungseinheit eines Gassensors nach den Ansprüchen 9-11 ausgeführt wird.

9. Ein Gassensor (10), umfassend:
eine Menge von Gassensorzellen (1) mit den gleichen Gaserkennungseigenschaften, jede Gassensorzelle (1) umfassend auf oder integriert in einem Substrat (14):
ein gassensitives Element (11),
eine Menge von Elektroden (16),
einen Heizer (15), um das zugeordnete gassensitive Element (11) zu heizen,
eine Steuerungseinheit, die konfiguriert ist, die Heizer (15) der Menge der Gassensorzellen (1) einzeln zu steuern,
wobei die Steuerungseinheit konfiguriert ist, während eines ersten Zeitabschnitts (T1) im Leben des Gassensors (10) Gasmessungen mit einer ersten Gassensorzelle (FSC) der Menge durch Einschalten des zugeordneten Heizers (15), um das zugeordnete gassensitive Element (11) zu heizen, durchzuführen, und gleichzeitig den Heizer (15) von mindestens einer der anderen Gassensorzellen (OSC) der Menge ausgeschaltet zu lassen oder diesen Heizer nur für das Durchführen einer Referenz-Gasmessung mit einer niedrigeren Rate als das Durchführen der Gasmessung durch die erste Gassensorzelle,
wobei als Reaktion auf einen Auslöseevent (TR1) die Steuerungseinheit konfiguriert ist, während eines zweiten Zeitabschnitts (T2) im Leben des Gassensors (10) nach dem ersten Zeitabschnitt (T1) Gasmessungen durchzuführen mit den anderen Gassensorzellen (OSC) durch Einschalten des zugeordneten Heizers (15), um das zugeordnete gassensitive Element (11) zu heizen (11), und gleichzeitig den Heizer (15) der ersten Gassensorzelle (FSC) ausgeschaltet zu lassen oder diesen Heizer nur für das Durchführen von einer Wiederherstellungs-Gasmessung mit einer niedrigeren Rate als das Durchführen der Gasmessung durch die andere Gassensorzelle,
wobei die Steuerungseinheit konfiguriert ist, den Auslöser (TR1) in Abhängigkeit von einem oder mehreren von einem Wert des gemessenen elektrischen Widerstands (R1) und eine Änderung im gemessenen elektrischen Widerstand (ΔR1) beim Einschalten des Heizers (15) überschreitet eine zugeordnete Schwelle (15).

10. Gassensor (10) nach Anspruch 9,
Wobei jede Gassensorzelle (1) der Menge umfasst das gleiche gassensitive Element (11), und
wobei jede Gassensorzelle (1) der Menge ein Metalloxid-Material umfasst.

11. Gassensor (10) nach Anspruch 9 oder Anspruch 10,
wobei das Substrat (14) ein gemeinsames Substrat für alle Gassensorzellen (1) der Menge ist,
umfassend eine Membran (13), die separate Teile (131, 132, 133) umfasst, die jede eine gemeinsame Aussparung (12) im gemeinsamen Substrat (14) überspannen (12),
wobei jeder Teil (131, 132, 133) einen der Heizer (15) und das zugeordnete gassensitive Element (11) trägt.

## Revendications

1. Procédé de fonctionnement d'un capteur de gaz, le capteur de gaz (10) comprenant un ensemble de cellules de détection de gaz (1) ayant les mêmes propriétés de détection de gaz, chaque cellule de détection de gaz (1) comprenant sur ou intégré dans un substrat (14) un élément sensible au gaz (11), un ensemble d'électrodes (16), un élément chauffant (15) pour chauffer l'élément sensible au gaz correspondant, et une unité de commande (4) pour contrôler les éléments chauffants des cellules individuelles de détection de gaz et effectuer les étapes de la méthode ci-dessous, la méthode comprenant
au cours d'une première période (T1) de la durée de vie du capteur de gaz (10), effectuer des mesures de gaz avec une première cellule de détection de gaz (FSC) de l'ensemble en activant l'élément chauffant correspondant (15) pour chauffer l'élément sensible correspondant (11), tout en laissant l'élément chauffant (15) d'une autre cellule de détection de gaz (OSC) de l'ensemble inactif ou en activant cet élément chauffant uniquement pour effectuer une mesure de gaz de référence à un taux inférieur à la réalisation de mesures de gaz par la première cellule de détection de gaz,
en réponse à un événement déclencheur (TR1), effectuer des mesures de gaz pendant une deuxième période (T2) durant la durée de vie du capteur de gaz (10) suivant la première période (T1) avec l'autre cellule de détection de gaz (OSC) en activant l'élément chauffant correspondant (15) pour chauffer l'élément sensible correspondant (11), tout en laissant l'élément chauffant (15) de la première cellule de détection de gaz (FSC) inactif ou en activant cet élément chauffant uniquement pour effectuer une mesure de gaz de référence à une fréquence inférieure aux mesures de gaz effectuées par l'autre cellule de détection de gaz,
dans lequel l'effectuation d'une mesure de gaz comprend la mesure d'une résistance électrique (R11) de la cellule de détection de gaz concernée (1) pendant ou après avoir activé l'élément chauffant correspondant (15), et
dans lequel l'évènement déclencheur (TR1) est généré par l'unité de commande (4) en fonction d'une ou de plusieurs valeurs de la résistance électrique (R1) mesurée et d'une modification de la valeur de résistance électrique mesurée (ΔR1) dépassant un seuil attribué en réponse à l'activation de l'élément chauffant (15).

2. Procédé selon la revendication 1, comprenant
générer l'évènement déclencheur (TR1) en fonction d'une ou plusieurs valeurs de résistance électrique (R11) mesurées au cours de la dernière période (T1), et en particulier générer l'évènement déclencheur (TR1) en fonction d'un ou plusieurs de:
une moyenne des valeurs de résistance électrique (AVG(Rll)) mesurées jusqu'à présent au cours de la première période (T1) est inférieure ou supérieure à un seuil attribué (TAVG);
une moyenne mobile des valeurs de résistance électrique mesurées au cours de la première période (T1) est inférieure ou supérieure à un seuil attribué;
un écart-type des valeurs de résistance électrique (ET(R11)) mesurées jusqu'à présent au cours de la première période (T1) est inférieur ou supérieur à un seuil attribué (DST);
un écart-type mobile des valeurs de résistance électrique mesurées au cours de la première période (T1) est inférieur ou supérieur à un seuil attribué;
une partie de changements à haute fréquence dans les valeurs de résistance électrique mesurées.

3. Procédé selon la revendication 1, comprenant
générer l'évènement déclencheur (TR1) en fonction d'un ou plusieurs de:
la variation de la valeur de résistance électrique mesurée (ΔR11) en réponse à l'activation de l'élément chauffant (15) dépasse un seuil attribué (TDELTA);
une période dans le temps (TTD) pour que la valeur de résistance électrique mesurée (R11) atteigne une valeur cible en réponse à l'activation de l'élément chauffant (15) dépasse un seuil attribué (THTG).

4. Procédé selon l'une quelconque des revendications précédentes, comprenant
au cours de la première période (T1), laisser inactifs les éléments chauffants (15) d'au moins deux autres cellules de détection de gaz (OSC) de l'ensemble,
au cours de la deuxième période (T2), effectuer des mesures de gaz avec une première (OSC1) d'au moins deux autres cellules de détection de gaz (OSC), tout en laissant inactif l'élément chauffant (15) d'une seconde (OSC2) d'au moins deux autres cellules de détection de gaz (OSC),
en réponse à un autre événement déclencheur (TR2), effectuer des mesures de gaz pendant une troisième période (T3) pendant la durée de vie du capteur de gaz (10) suivant la deuxième période (T2) avec la deuxième (OSC2) des au moins deux autres cellules de détection de gaz (OSC), tout en laissant l'élément chauffant (15) de la première cellule de détection de gaz (FSC) et la première (OSC1) des au moins deux autres cellules de détection de gaz (OSC) inactif.

5. Procédé selon l'une quelconque des revendications précédentes, comprenant, en réponse à un autre événement déclencheur (TR3), effectuer des mesures de gaz, pendant une période ultérieure de la durée de vie du capteur de gaz (10) au moins après la deuxième période (T2), à nouveau avec la première cellule de capteur de gaz (FSC), tout en laissant l'élément chauffant (15) d'au moins une autre cellule de détection de gaz (OSC) inactif.

6. Procédé selon l'une quelconque des revendications précédentes, comprenant, pendant la durée de vie du capteur de gaz (10),
laisser inactif l'élément chauffant (15) d'une troisième cellule de détection de gaz (TSC) de l'ensemble, sauf pour effectuer des mesures de gaz de référence, et
comparer un résultat de la mesure de gaz effectuée avec l'une des premières cellules de détection de gaz (FSC) ou au moins une autre cellule de détection de gaz (OSC) avec un résultat de la mesure de gaz de référence effectuée avec la troisième cellule de détection de gaz (TSC).

7. Procédé selon la revendication 6, dans lequel
l'effectuation d'une mesure de gaz comprend la mesure d'une résistance électrique (R11) de la cellule de détection de gaz concernée (FSC, OSC) pendant ou après avoir activé l'élément chauffant correspondant (15), et
comprenant la génération de l'évènement déclencheur (TR1) en fonction d'une valeur de résistance électrique mesurée lors de la mesure du gaz et en fonction d'une valeur de résistance électrique de référence mesurée lors de la mesure du gaz de référence, et
en particulier la génération de l'évènement déclencheur (TR1) en fonction d'un ou plusieurs des :
la valeur mesurée s'écarte de la valeur de référence mesurée de plus d'une marge attribuée;
une modification de la valeur mesurée en réponse à l'activation de l'élément chauffant correspondant s'écarte d'une modification de la valeur de référence mesurée en réponse à l'activation de l'élément chauffant correspondant de plus d'une marge attribuée;
une période dans le temps pour que la valeur de résistance électrique mesurée atteigne une valeur cible en réponse à l'activation de l'élément chauffant correspondant s'écarte d'une période dans le temps pour que la valeur de référence mesurée atteigne la valeur cible en réponse à l'activation du l'élément chauffant correspondant de plus d'une marge attribuée.

8. Élément lisible par ordinateur comprenant des moyens de code lisible par ordinateur pour effectuer les étapes d'un procédé selon l'une quelconque des revendications précédentes lorsqu'il est exécuté sur une unité de commande d'un capteur de gaz selon les revendications 9-11.

9. Un capteur de gaz (10), comprenant:
un ensemble de cellules de détection de gaz (1) ayant les mêmes propriétés de détection de gaz, chaque cellule de détection de gaz (1) comprenant sur ou intégrée dans un substrat (14):
un élément sensible aux gaz (11),
un ensemble d'électrodes (16),
un élément chauffant (15) pour chauffer l'élément sensible au gaz correspondant (11),
une unité de commande configurée pour contrôler individuellement les éléments chauffants (15) de l'ensemble des cellules de détection de gaz (1),
dans lequel l'unité de commande est configurée pour, pendant une première période (T1) de la durée de vie du capteur de gaz (10), effectuer des mesures de gaz avec une première cellule de détection de gaz (FSC) de l'ensemble en activant l'élément chauffant correspondant (15) pour chauffer l'élément sensible correspondant (11) tout en laissant l'élément chauffant (15) d'au moins une autre cellule de détection de gaz (OSC) de l'ensemble inactif ou en activant cet élément chauffant uniquement pour effectuer une mesure de gaz de référence à une fréquence inférieure aux mesures de gaz effectuées par la première cellule de détection de gaz,
dans lequel, en réponse à un événement déclencheur (TR1), l'unité de commande est configurée pour, pendant une deuxième période (T2) de la durée de vie du capteur de gaz (10) suivant la première période (T1), effectuer des mesures de gaz avec l'autre cellule de détection de gaz (OSC) en activant l'élément chauffant correspondant (15) pour chauffer l'élément sensible correspondant (11), tout en laissant l'élément chauffant (15) de la première cellule de détection de gaz (FSC) inactif ou en activant cet élément chauffant uniquement pour effectuer une mesure de gaz de référence à une fréquence inférieure aux mesures de gaz effectuées par l'autre cellule de détection de gaz,
dans lequel l'unité de commande est configurée pour générer l'événement déclencheur (TR1) en fonction d'une ou de plusieurs valeurs de la résistance électrique (R1) mesurée et d'une modification de la valeur de résistance électrique mesurée (ΔR1) dépassant un seuil attribué en réponse à l'activation de l'élément chauffant (15).

10. Capteur de gaz (10) selon la revendication 9,
dans laquelle chaque cellule de détection de gaz (1) de l'ensemble comprend le même élément sensible au gaz (11), et
dans laquelle chaque cellule de détection de gaz (1) de l'ensemble comprend un matériau d'oxyde métallique.

11. Capteur de gaz (10) selon la revendication 9 ou la revendication 10,
dans lequel le substrat (14) est un substrat commun à toutes les cellules de détection de gaz (1) de l'ensemble,
comprenant une membrane (13), la membrane (13) comprenant des sous-parties séparées (131, 132, 133) couvrant chacune un renfoncement commun (12) dans le substrat commun,
dans laquelle chaque sous-portion (131, 132, 133) supporte l'un des éléments chauffants (15) et l'élément sensible correspondant (11).
